# FASCICULE DE BREVET EUROPEEN

(11) **EP 0 788 549 B1**
(45) Date de publication et mention de la délivrance du brevet: **13.10.2004**
(21) Numéro de dépôt: 95902830.9
(22) Date de dépôt: 05.12.1994
(51) Int. Cl.: C12N 15/76

(54) **SEQUENCES D'ACIDES NUCLEIQUES REGULATRICES ET UTILISATIONS**
REGULIERENDE NUKLEINSAEURESEQUENZEN UND VERWENDUNGEN
REGULATORY NUCLEIC ACID SEQUENCES AND USES THEREOF

(30) Priorité: 08.12.1993 FR 9314701
(43) Date de publication de la demande: 13.08.1997
(73) Titulaire: Aventis Pharma S.A., 92160 Antony (FR)
(72) Inventeur: FRIEDMANN, Annick, F-91190 Villiers-le-Bacle (FR); GUERINEAU, Michel, F-75006 Paris (FR); HAGEGE, Juliette, F-91400 Orsay (FR); PERNODET, Jean-Luc, F-94230 Cachan (FR); SEZONOV, Guénnady, F-75006 Paris (FR)
(74) Mandataire: Le Pennec, Magali
(86) Numéro de dépôt international: PCT/FR1994/001413
(87) Numéro de publication internationale: WO 1995/016046

(56) Documents cités:
- EP-A- 0 350 341
- PLASMID, vol.25, no.1, Janvier 1991 pages 40 - 52 T. SMOKVINA ET AL 'Functional analysis of the Streptomyces ambofaciens element pSAM2'
- MOLECULAR AND GENERAL GENETICS, vol.198, no.1, 1984 pages 35 - 41 J.L. PERNODET ET AL 'Plasmids in different strains of Streptomyces ambofaciens : Free and integrated form of plasmid pSAM2' cité dans la demande
- JOURNAL OF BACTERIOLOGY, vol.175, no.17, Septembre 1993 pages 5529 - 5538 J. HAG GE ET AL 'Transfer functions of the conjugative integrating element pSAM2 from Streptomyces ambofaciens : Characterization of a kil-kor system associated with transfer'
- PLASMID, vol.31, no.2, Février 1994 pages 166 - 183 J. HAG GE ET AL 'Identification of a gene encoding the replication initiator protein of the Streptomyces integrating element , pSAM2'

## Description

La présente invention concerne une nouvelle séquence nucléique, des vecteurs pour son expression, et son utilisation dans des procédés de fermentation chez les Actinomycetes.

Les Actinomycetes sont sont des bactéries Gram (+) filamenteuses et ramifiées. Parmi les Actinomycetes, les Streptomycetes représentent la famille la plus importante. Les Streptomycetes sont des bactéries filamenteuses, sporulantes et vivant naturellement dans le sol dans des conditions de stricte aérobiose.

Les Actinomycetes, et notamment les Streptomycetes, présentent un grand intérêt sur le plan industriel. En particulier, ils présentent la particularité de produire une grande variété de métabolites secondaires (Demain, Biology of Actinomycetes 88, Okami (Eds), Tokyo, Japan scientific societies press, 1988, p. 19-25). Ces métabolites peuvent avoir des structures et des propriétés biologiques très différentes (herbicides, anticancéreux, antihelminthiques, anabolisants, antibiotiques, etc). Les plus connus de ces métabolites sont les antibiotiques (substances chimiques produites par un organisme et ayant un effet néfaste sur d'autres organismes). Les Streptomyces sont actuellement à l'origine de 70% des antibiotiques produits industriellement. La diversité structurale des antibiotiques synthétisés ne se retrouve dans aucun autre genre bactérien. Ainsi, presque tous les types de structure sont représentés : les β lactamines (ex. l'ampicilline), les antibiotiques polypeptidiques (ex. streptogramines), les antibiotiques aminoglycosidiques (ex. streptomycine, kanamycine, etc), les macrolides (ex. erythromycine, spiramycine, etc), ou encore les cyclines (ex. tétracycline), etc.

Pour ces raison, il est particulièrement intéressant de pouvoir disposer d'outils (vecteurs, promoteurs, etc) permettant de manipuler génétiquement ces microorganismes. De tels outils permettraient en effet de modifier les niveaux de synthèse de ces métabolites, ou de préparer des intermédiaires de synthèse ou des dérivés de ces métabolites, etc. De tels outils permettraient également de faire fabriquer par ces microorganismes des produits recombinants, notamment des protéines hétérologues, selon les techniques du génie génétique.

A cet égard, la demande de brevet n° EP 350 341 décrit des vecteurs dérivés du plasmide pSAM2 ayant des propriétés très intéressantes. Ainsi, ces vecteurs sont capables de s'intégrer de manière site-spécifique dans le génome des Actinomycetes, possèdent un large spectre d'hôte et une stabilité élevée. Par ailleurs, ils peuvent être utilisés pour le transfert et l'expression d'acides nucléiques dans les Actinomycetes. Toutefois, ces vecteurs présentent certains inconvénients qui résident notamment dans leur faible nombre de copies par cellules, et dans l'absence de moyens de contrôle du nombre de copies. Ainsi, pSAM2 et ses dérivés s'intègrent généralement à raison d'une seule copie par chromosome.

La présente invention apporte une solution à ce problème, en fournissant des outils capables d'améliorer les conditions d'utilisation industrielle de vecteurs dérivés de pSAM2. La présente invention décrit en effet un gène dont le produit d'expression conduit à l'apparition, à partir de formes intégrées, de formes libres réplicatives du plasmide pSAM2 ou de vecteurs dérivés de celui-ci. Ceci a pour effet d'augmenter le nombre de copies de pSAM2 ou de ses dérivés car les formes libres sont présentes à un nombre élevé de copies par cellule.

La présente invention décrit aussi des cassettes d'expression de ce gène, des vecteurs le contenant, et leur utilisation pour induire l'apparition de copies libres de pSAM2 ou de vecteurs intégratifs dérivés de celui-ci.

La demanderesse a en effet isolé, séquencé et caractérisé une région du plasmide pSAM2 capable d'induire l'apparition de copies libres réplicatives de pSAM2 ou de ses dérivés. La demanderesse a également montré que cette région pouvait être utilisée en cis (sur le même vecteur) ou en trans (pas sur le même vecteur) pour agir sur pSAM2 ou ses dérivés. La séquence de cette région est présentée sur la séquence SEQ ID n° 1. Plus précisément, cette région et son rôle fonctionel ont été mis en évidence par l'étude de variants du plasmide pSAM2 : d'une part pSAM2(B2), provenant de S.ambofaciens ATCC 15154 pour lequel aucune forme libre n'est observée, d'autre part pSAM2(B3) et pSAM2(B4) provenant d'autres S. ambofaciens, pour lesquels la forme libre est observée avec la forme intégrée. Cette étude a permis de caractériser deux mutations ponctuelles différentes (celles de pSAM2(B3) et celle de pSAM2(B4)) localisées toutes deux dans la région promotrice contrôlant l'expression d'un gène de pSAM2 (Cf SEQ ID n° 1). Ces mutations conduisent à l'apparition de la forme libre de pSAM2(B3) et de pSAM2(B4). Ce gène a ensuite été cloné et séquence, et sa capacité à provoquer l'apparition de la forme libre de plasmides dérivés de pSAM2 à partir de la copie intégrée a été démontrée.

Un premier objet de l'invention réside donc dans une séquence d'acide nucléique comprenant tout ou partie de la séquence SEQ ID n° 1 ou d'un variant de celle-ci résultant de la dégénérescence du code génétique et ayant la capacité d'induire des forme libres réplicatives du plasmide pSAM2 dans les Actinomycetes.

Au sens de la présente invention, le terme variant désigne toute séquence différant de la séquence SEQ ID n°1 en raison de la dégénérescence du code génétique, ainsi que toute séquence hybridant avec ces séquences ou des fragments de celles-ci et dont le produit possède l'activité indiquée. Ces variants peuvent être obtenus par toute technique connue de l'homme du métier, à partir de SEQ ID n° 1, notamment, mutation, délétion, substitution, addition, hybridation, etc. Les hybridations peuvent être réalisées dans des conditions de stringence conventionnelles (Maniatis et al., Cf techniques générales de biologie moléculaire), ou, de préférence, dans des conditions de stringence élevées. La capacité des variants d'induire l'apparition de formes libres réplicatives de pSAM2 ou de ses dérivés peut être déterminée sur une souche d'Actinomycète contenant un tel plasmide intégré (par exemple sur la souche ATCC 15154), en tranfectant ledit variant dans la souche, dans des conditions permettant son expression, et en vérifiant l'apparition de formes libres (Cf exemples).

Un autre objet de l'invention concerne toute cassette d'expression de la séquence SEQ ID n° 1 ou d'un variant de celle-ci tel que défini ci-avant, comprenant ladite séquence ou variant sous controle d'un promoteur constitutif ou régulé.

L'utilisation d'un promoteur constitutif est particulièrement avantageuse lorsque la cassette est utilisée en trans pour induire des formes libres d'un plasmide intégré. Dans ce cas, les cellules contenant la forme intégrée du plasmide sont transfectées avec la cassette d'expression pour induire l'apparition de formes libres réplicatives, permettant par exemple d'isoler et/ou de purifier le plasmide.

L'utilisation d'un promoteur régulé est particulièrement avantageuse lorsque la cassette est utilisée en cis (sur le vecteur dérivé de pSAM2 lui-même), par exemple dans un procédé industriel de fermentation. Dans ce cas, toute la phase de prolifération et de croissance des cellules est effectuée sans expression du gène de l'invention, c'est-à-dire avec une seule copie du plasmide par chromosome, et, pour la phase de production (d'un produit recombinant, d'un gène de biosynthèse ou de régulation de la synthèse d'un métabolite, etc), le promoteur régulé est induit, entrainant l'apparition de plusieurs copies libres du plasmide et ainsi une activité de production accrue. Ce mode de mise en oeuvre est particulièrement avantageux lorsque le vecteur dérivé de pSAM2 porte des séquences hétérologues dont la présence en plusieurs nombre de copies peut être toxique aux cellules et/ou affecter leur croissance.

Parmi les promoteurs constitutifs utilisables dans le cadre de la présente invention, on peut citer plus particulièrement tout promoteur constitutif fonctionnel dans les Actinomycètes, tel que par exemple le promoteur du gène ermE ou un variant de celui-ci (Bibb et al., Gene 41 (1986) E357), le promoteur pl4 du phage 119 de S.ghanaensis (Labes et al., Sixth DFGWT/AFAST, 27-30/11/92), ou tout fragment contenant une région promotrice d'un opéron ribosomique de S.ambofaciens (Pemodet et al., Gene 79 (1989) 33).

Parmi les promoteurs régulables utilisables dans le cadre de la présente invention, on peut citer plus particulièrement tout promoteur régulable fonctionnel dans les Actinomycètes. Il peut s'agir de promoteurs induits spécifiquement par un agent introduit dans le milieu de culture, tel que par exemple le promoteur tipA inductible par le thiostrepton (Murakami et al., J. Bactt. 171 (1989) 1459) ou des promoteurs thermoinductibles comme celui des gènes groEL par exemple (Mazodier et al., J. Bact. 173 (1991) 7382). Il peut également s'agir d'un promoteur d'actinomycètes spécifiquement actif dans les phases tardives du cycle de prolifération des actinomycètes, tel que par exemple certains promoteurs de gènes du métabolisme secondaire (gènes de production d'antibiotiques notamment).

Un autre objet de l'invention concerne l'utilisation des séquences de l'invention telles que définies ci-avant, ou des cassettes les contenant, pour induire l'apparition de copies libres de vecteurs dérivés de pSAM2 intégrés dans un Actinomycète.

Comme indiqué plus haut, cette utilisation peut être effectuée en cis (le gène ou la cassette étant portés par le vecteur intégratif dérivé de pSAM2) ou en trans (le gène ou la cassette étant sur un autre vecteur ou même introduits directement tels quels).

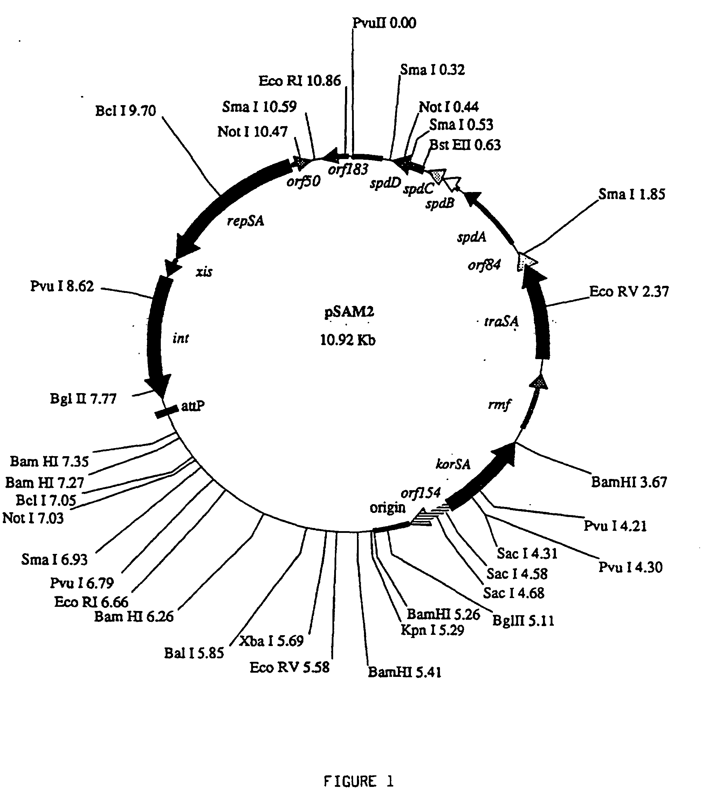

Les vecteurs intégratifs dérivés de pSAM2 tels que mentionnés ci-dessus sont des vecteurs comprenant au moins les éléments de pSAM2 nécessaires à l'intégration, à l'excision et à la réplication. Plus particulièrement, ces vecteurs comprennent donc au moins les régions attP et int telles que décrites dans la demande EP 350 341, le gène Xis, le gène repSA et l'origine de réplication (ori). Ces différentes régions, sont représentées sur la carte du plasmide pSAM2 donnée sur la figure 1, qui mentionne également certains sites de restriction permettant d'extraire ces régions.

Avantageusement, les vecteurs intégratifs dérivés de pSAM2 comprennent également une séquence d'ADN recombinante codant pour un produit recherché. Il peut s'agir d'un peptide, polypeptide ou protéine présentant un intérêt pharmaceutique ou agroalimentaire. Dans ce cas, le système de l'invention permet d'augmenter le nombre de copies de cette séquence par cellule et donc d'augmenter les niveaux de production de ce produit et ainsi d'augmenter les rendements du procédé de préparation. Le produit recherché peut également être un peptide, polypeptide ou protéine intervenant dans la biosynthèse (synthèse, dégradation, transport ou régulation) d'un métabolite par la souche d'Actinomycète concernée. Dans ce cas, lé système de l'invention permet d'augmenter le nombre de copies de cette séquence par cellule et donc d'augmenter les niveaux de production de ce produit et ainsi soit d'augmenter les niveaux de production du métabolite, soit de bloquer la biosynthèse du métabolite, soit de produire des dérivés du métabolite.

Les séquences de l'invention peuvent ainsi être utilisées dans tout Actinomycète dans le génome duquel les vecteurs pSAM2 ou ses dérivés sont capables de s'intégrer. En particulier, elles peuvent être utilisées dans des procédés de fermentation impliquant des souches de Streptomyces, de mycobactéries, etc. A titre d'exemple, on peut citer les souches de S. pristinaespiralis (ATCC 25486), S. antibioticus (DSM 40868), S. bikiniensis (ATCC 11062), S. parvulus (ATCC 12434), S. glaucescens (ETH 22794), S. actuosus (ATCC25421), S. coelicolor (A3(2)), S. ambofaciens, S. lividans, S. griseofuscus, S. limosus, etc (Voir également Smokvina et al., Applications of the integrated plasmid pSAM2, GIN490, Proceedings, Vol. 1, p.403-407).

Des vecteurs dérivés de pSAM2 comportant les éléments décrits ci-dessus peuvent être construits par l'homme du métier, sur la base de ses connaissances générales et des enseignements de la présente demande (Cf également les techniques générales de biologie moléculaire).

Les séquences de l'invention sont tout particulièrement adaptées à une utilisation dans un procédé industriel de production d'antibiotiques (spiramycine, streptogramines, β lactames, etc dont les gènes sont décrits dans les demandes EP 346 000 et PCT/FR93/00923 notamment).

La présente invention sera plus complètement décrite à l'aide des exemples qui suivent, qui doivent être considérés comme illustratifs et non limitatifs.

**Légende des figures :**
Figure 1 : Carte de restriction du plasmide pSAM2
Figure 2 : Carte de restriction du vecteur pOS531
Figure 3 : Carte de restriction du vecteur pOS532
Figure 4 : Carte de restriction du vecteur pOS541
Figure 5 : Carte de restriction du vecteur pOS544
Figure 6 : Carte de restriction du vecteur pOS666. Ce vecteur est construit à partir du plasmide pPM927 (Smokvina et al. gene 94 (1990) 53) par insertion du gène rmf (également désingé pra) et de l'origine de réplication de pSAM2 : tsr : gène de résistance au thiostrepton; stm/spc : gène de résistance à la streptomycine/spectinomycine; oriR : origine de réplication E. coli; oripSAM2 : origine de réplication de pSAM2; ter : terminateur de transcription.
Figure 7 : Activité des vecteurs.

### Techniques générales de biologie moléculaire

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropanol, la transformation dans Escherichia coli, etc ... sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, New York, 1987].

Les plasmides de type pBR322, pUC et les phages de la série M13 sont d'origine commerciale (Bethesda Research Laboratories).

Pour les ligatures, les fragments d'ADN peuvent être séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.

Le remplissage des extrémités 5' proéminentes peut être effectué par le fragment de Klenow de l'ADN Polymérase I d'E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.

La mutagénèse dirigée in vitro par oligodéoxynucléotides synthétiques peut être effectuée selon la méthode développée par Taylor et al. [Nucleic Acids Res. 13 (1985) 8749-8764] en utilisant le kit distribué par Amersham.

L'amplification enzymatique de fragments d'ADN par la technique dite de PCR [Polymérase-catalyzed Chain Reaction, Saiki R.K. et al., Science 230 (1985) 1350-1354; Mullis K.B. et Faloona F.A., Meth. Enzym. 155 (1987) 335-350] peut être effectuée en utilisant un "DNA thermal cycler" (Perkin Elmer Cetus) selon les spécifications du fabricant.

La vérification des séquences nucléotidiques peut être effectuée par la méthode développée par Sanger et al. [Proc. Natl. Acad. Sci. USA, 74 (1977) 5463-5467] en utilisant le kit distribué par Amersham.

### Exemples

### Exemple 1 : Clonage et séquençage de la séquence SEQ ID n° 1

La forme libre de pSAM2 (B2) n'est pas observée dans S. lividans TK24 (Hopwood et al., J. Gen. Microbiol. 129 (1983) 2257), seule la forme intégrée est observée. Avec pSAM2 (B3) la forme libre du plasmide coexiste avec la forme intégrée. Des plasmides hybrides ont été construits à partir de pSAM2 (B3) dans lesquels différentes régions ont été remplacées par les régions équivalentes provenant de pSAM2 (B2). Ceci a permis de montrer que la mutation permettant à pSAM2 (B3) d'exister sous forme libre était localisée dans un fragment de restriction KpnI de 2 kb. La séquence de ce fragment KpnI a été déterminée pour pSAM2 (B2) et pSAM2 (B3). Un seul nucléotide diffère entre ces deux séquences : une paire G/C dans pSAM2 (B2) est remplacée par une paire A/T dans pAM2 (B3). L'analyse de la séquence a montré que cette mutation se trouvait en amont d'une phase ouverte de lecture qui se prolongeait plus loin que le site KpnI. La séquence de cette phase ouverte de lecture a été déterminée, elle est présentée sur la séquence SEQ ID n°1. Cette phase ouverte de lecture, désignée rmf (ou pra), est située entre les gènes korSA et traSA. La mutation fait disparaître un site de reconnaissance pour l'enzyme de restriction ApaLI (site de reconnaissance : 5'GTGCAC 3'). Ce site est présent chez pSAM2 (B2) mais absent chez pSAM2 (B3).

La séquence SEQ ID n°1 comprend également 100 pb en amont de la région codante, comprenant une partie du promoteur (résidus 1 à 101) et la région 5' non codant mais transcrite (résidus 102 à 154), portant notamment le site de liaison des ribosomes (RBS).

Dans le mutant pSAM2 (B4), pour lequel la forme libre peut être observée, le site ApaLI est encore présent, indiquant que la mutation n'est pas localisée au même endroit que dans pSAM2 (B3). La séquence de pSAM2 (B4) pour la région correspondante a montré que les séquences de pSAM2 (B2) et de pSAM2 (B4) différaient par un nucléotide. Cette mutation était localisée 8 nucléotides en amont de la mutation détectée dans pSAM2 (B3). Ainsi, dans deux cas indépendants, la présence de la forme libre de pSAM2 (B3) et pSAM2 (B4), qui coexiste avec la forme intégrée, est due à une mutation ponctuelle en amont d'un gène de pSAM2. Ces deux mutations indépendantes sont toutes deux situées dans une région dont les expériences d'analyses des ARN messagers ont montré qu'elle constituait le promoteur de ce gène.

### Exemple 2 Construction de cassettes et vecteurs d'expression de la séquence SEQ ID n°1

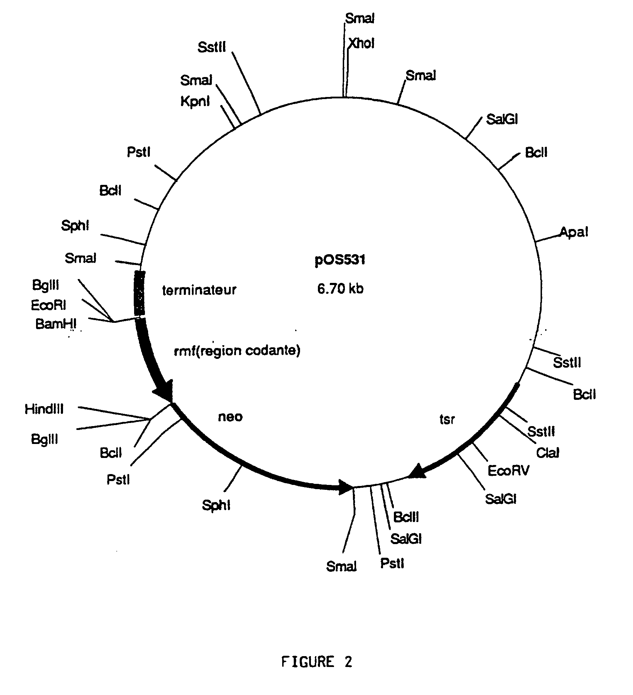

Plusieurs constructions ont été réalisées en utilisant les promoteurs et plasmides suivants :
- Promoteur ermE modifié (ermE*). Le promoteur ermE modifié utilisé correspond au promoteur décrit par Bibb et al précitée possédant une délétion de 3 nucléotides (bases 252-254, figure 2 de Bibb et al. précitée). Ce promoteur modifié donne une expression forte et constitutive. Il a été isolé sous forme d'un fragment KpnI-BamHI de 275 pb.
- Promoteur tipA : Ce promoteur (Murakami et al. précitée) est présent dans le plasmide pPM927 (Smokvina et al., Gene 94 (1990) 53). Il est induit spécifiquement en présence de thiostrepton.
- Plasmides pU486 et pU487. Ces deux plasmides ont été décrits par Ward et al. (Mol. Gen. Genet. 203 (1986) 468). Ces plasmides seuls n'ont pas d'influence sur le statut (libre ou intégré) de pSAM2.

### 2.1. Construction du vecteur pOS531

Le vecteur pOS531 a été construit par clonage de la partie codante du gène rmf (résidus 155 à 505 de la séquence SEQ ID n° 1) aux sites BamHI-HindIII du plasmide pIJ487. Ce vecteur porte donc le gène rmf nu (sans signal d'expression ni région 5' non codante). Une carte de ce vecteur est donnée sur la figure 2.

### 2.2. Construction du vecteur pOS532

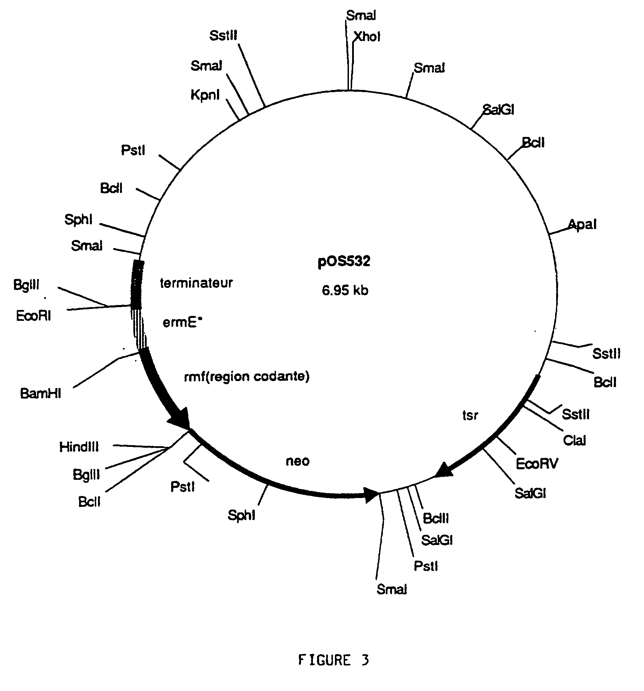

Le vecteur pOS532 a été construit par clonage du fragment de 275 pb portant le promoteur ermE modifié aux sites EcoRI-BamHI du vecteur pOS531. Ce vecteur porte donc le gène rmf nu sous contrôle du promoteur ermE modifié (figure 3).

### 2.3. Construction du vecteur pOS541

Le vecteur pOS541 a été construit en 2 étapes :
- clonage d'un fragment comportant la partie codante du gène rmf et la région 5' non codante (résidus 102 à 505 de la séquence SEQ ID n° 1) aux sites BamHI-HindIII du plasmide pIJ487,
- addition du fragment de 275 pb portant le promoteur ermE modifié aux sites EcoRI-BamHI du vecteur obtenu ci-dessus.

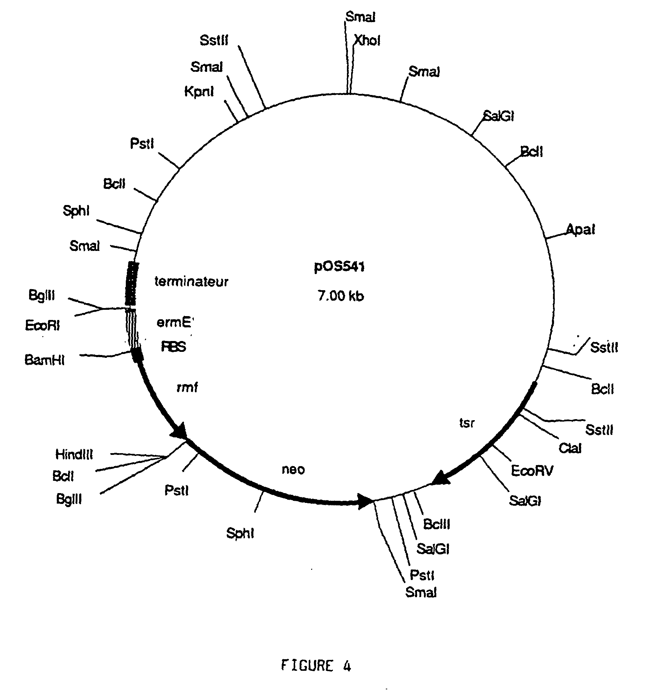

Ce vecteur porte donc le gène rmf pourvu de sa région 5' non codante, sous contrôle du promoteur ermE modifié (figure 4).

### 2.4. Construction du vecteur pOS544

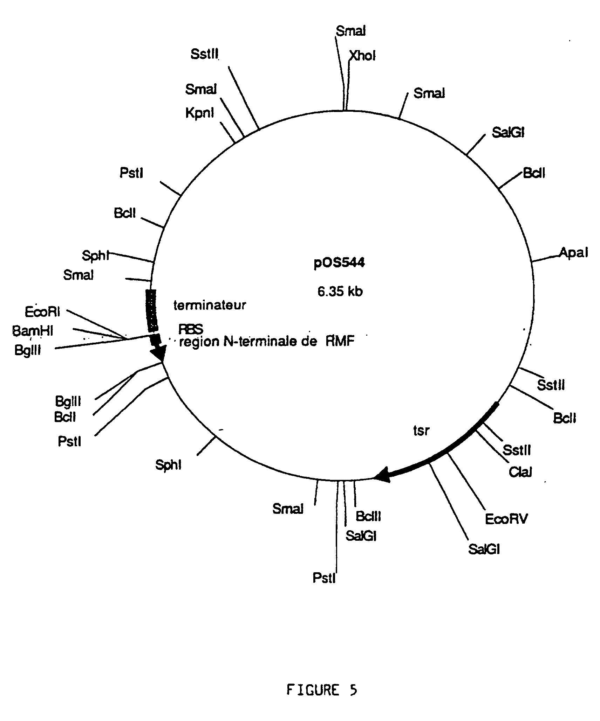

Le vecteur pOS544 porte un gène rmf délété dans sa partie 3' (les résidus 102 à 276 de la séquence SEQ ID n° 1 sont présents). Il a été construit par clonage de cette région sous forme d'un fragment BamHï-BspHI (extrémité BspHI traitée par le fragment de Klenow de l'ADN polymérase 1 d'E. coli) dans les sites BamHI HindIII du plasmide pIJ487 (extrémité HindIII traitée par le fragment de Klenow de l'ADN polyinérase I d'E. coli). Le vecteur pOS544 porte donc la partie 5' du gène rmf (région 5' non codante + région 5' codante), mais dépourvue de son promoteur. Une carte de ce vecteur est donnée sur la figure 5.

### Exemple 3 Apparition de formes libres réplicatives de vecteurs intégratifs dérivés de pSAM2 due au produit d'expression du gène rmf

Cet exemple montre que la surexpression du gène SEQ ID n° 1 peut provoquer l'apparition sous forme libre d'une copie intégrée de pSAM2.
3.1. Une preuve directe du rôle d'activateur de la réplication que joue rmf peut être obtenue en provoquant, par expression de rmf; l'apparition sous forme libre d'une copie intégrée de pSAM2 (B2). Le fait que pSAM2 (B2) possède un site ApaLI supplémentaire par rapport à pSAM2 (B3) permet de vérifier simplement par observation du profil de digestion ApaLI que le plasmide libre est bien pSAM2 (B2).

3.2. L'activité de la séquence SEQ ID n° 1 a également été mise en évidence par transformation de S.lividans portant une copie intégrée de pSAM2(B2) avec les différents vecteurs décrits ci-dessus, puis recherche de formes libres. Pour cela, S. lividans contenant le plasmide pSAM2(B2) a été transformé par la technique des protoplastes par les différents vecteurs décrits dans l'exemple 2. L'ADN plasmidique ou l'ADN cellulaire total sont extraits à partir d'une culture en phase stationnaire des clones transformés. L'ADN plasmidique est digéré par l'enzyme ApaLI et les fragments de digestion séparés par électrophorèse en gel d'agarose. L'observation du profil de restriction permet de savoir si des copies libres de pSAM2(B2) sont présentes. Ces résultats sont ensuite confirmés par des expériences d'hybridation de l'ADN total avec une sonde pSAM2.

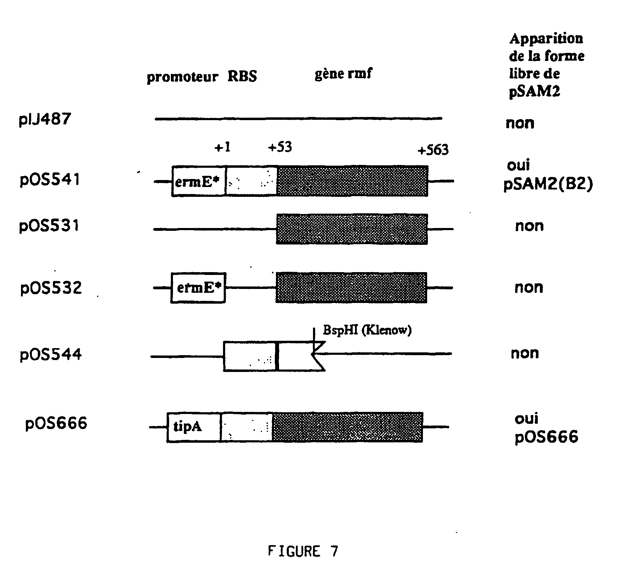

Les résultats obtenus sont présentés dans la figure 7.

Ces résultats montrent que pOS541, qui comporte le promoteur ermE modifié, la région non traduite en amont de rmf avec le site de fixation des ribosomes et la phase codant rmf, provoque l'apparition de formes libres réplicatives de pSAM2(B2). Comme attendu aucun effet n'est observé avec un vecteur ne comportant que la partie codante rmf (pOS531) ou avec un vecteur comportant ermE modifié et la partie codante de rmf, mais aucune région de fixation des ribosomes (pOS532). Aucun effet n'est observé avec un vecteur portant une délétion dans la partie 3' de rmf (pOS544).

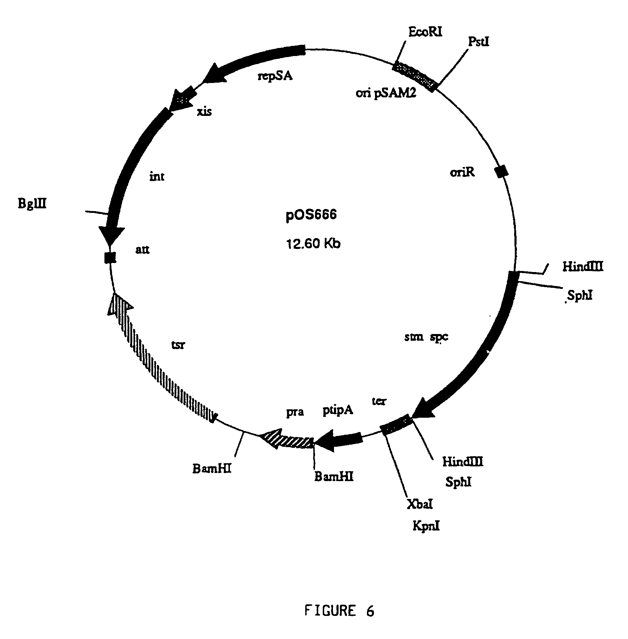

Le même type d'effet est obtenu quand la cassette permettant l'expression inductible de rmf est localisée sur le plasmide pour lequel on souhaite provoquer l'apparition de formes libres (vecteur pOS666, figure 6).

L'ensemble de ces résultats démontre bien que la séquence de l'invention est capable d'induire, en cis ou en trans, l'apparition de formes libres de vecteurs dérivés de pSAM2.

## Revendications

1. Molécule d'acide nucléique **caractérisée en ce qu'**elle est choisie parmi :
a) la séquence d'acide nucléique représentée par la SEQ ID N°1,
b) les fragments de cette séquence codant pour un polypeptide capable de permettre l'apparition, à partir de formes intégrées, de formes libres réplicatives du plasmide pSAM2 dans les Actinomycetes
c) les variant de cette séquence et résultant de la dégénérescence du code génétique et codant pour un polypeptide capable de permettre l'apparition, à partir de formes intégrées, de formes libres réplicatives du plasmide pSAM2 dans les Actinomycetes

2. Cassette d'expression comprenant une séquence selon la revendication 1 sous controle d'un promoteur constitutif ou régulé.

3. Cassette d'expression selon la revendication 2 **caractérisée en ce que** le promoteur constitutif est choisi parmi le promoteur du gène ermE ou un variant de celui-ci, le promoteur p14 du phage I19 de S.ghanaensis, ou tout fragment contenant une région promotrice d'un opéron ribosomique de S.ambofaciens.

4. Cassette d'expression selon la revendication 2 **caractérisée en ce que** le promoteur régulé est choisi parmi les promoteurs induits spécifiquement par un agent introduit dans le milieu de culture, tel que par exemple le promoteur tipA inductible par le thiostrepton ou des promoteurs thermoinductibles comme celui des gènes groEL.

5. Cassette d'expression selon la revendication 2 **caractérisée en ce que** le promoteur régulé est choisi parmi les promoteurs d'actinomycètes spécifiquement actifs dans les phases tardives du cycle de prolifération des actinomycètes, tel que par exemple les promoteurs de gènes du métabolisme secondaire (gènes de production d'antibiotiques notamment).

6. Utilisation d'une séquence ou cassette selon l'une des revendications précédentes pour induire l'apparition de copies libres de vecteurs dérivés de pSAM2 intégrés dans un actinomycète.

7. Utilisation selon la revendication 6 **caractérisée en ce que** la séquence ou la cassette sont portées par le vecteur intégratif dérivé de pSAM2.

8. Utilisation selon la revendication 6 **caractérisée en ce que** la séquence ou la cassette sont portées par un autre vecteur ou introduites directement telles quelles.

9. Utilisation selon les revendications 6 à 8 **caractérisée en ce que** l'Actinomycète est choisi parmi les Streptomyces et les mycobactéries.

## Claims

1. Nucleic acid molecule, **characterized in that** it is chosen from:
(a) the nucleic acid sequence represented by SEQ ID No.1,
(b) the fragments of this sequence encoding a polypeptide capable of allowing the appearance, from integrated forms, of replicative free forms of the plasmid pSAM2 in actinomycetes,
(c) the variants of this sequence which result from the degeneracy of the genetic code and which encode a polypeptide capable of allowing the appearance, from integrated forms, of replicative free forms of the plasmid pSAM2 in actinomycetes.

2. Expression cassette comprising a sequence according to Claim 1 under the control of a constitutive or regulated promoter.

3. Expression cassette according to Claim 2, **characterized in that** the constitutive promoter is chosen from the promoter of the ermE gene or a variant of the latter, the p14 promoter of phage 119 of S. ghanaensis, or any fragment containing a promoter region of a ribosomal operon of S. ambofaciens.

4. Expression cassette according to Claim 2, **characterized in that** the regulated promoter is chosen from promoters induced specifically by an agent introduced into the culture medium, such as, for example, the thiostrepton-inducible promoter tipA, or thermoinducible promoters such as that of the groEL genes.

5. Expression cassette according to Claim 2, **characterized in that** the regulated promoter is chosen from actinomycete promoters which are specifically active in the late phases of the proliferation cycle of actinomycetes, such as, for example, promoters of genes of the secondary metabolism (genes for the production of antibiotics, in particular).

6. Use of a sequence or cassette according to one of the preceding claims, for inducing the appearance of free copies of vectors which are derived from pSAM2 and integrated in an actinomycete.

7. Use according to Claim 6, **characterized in that** the sequence or cassette is carried by the integrative vector derived from pSAM2.

8. Use according to Claim 6, **characterized in that** the sequence or cassette is carried by another vector or introduced directly as such.

9. Use according to Claims 6 to 8, **characterized in that** the actinomycete is chosen from Streptomyces and mycobacteria.

## Patentansprüche

1. Nukleinsäuremolekül, **dadurch gekennzeichnet, dass** es ausgewählt ist unter:
a) der durch die SEQ ID NO:1 dargestellten Nukleinsäurefrequenz,
b) den Fragmenten dieser Frequenz, kodierend für ein Polypeptid, das aus integrierten Formen das Auftreten von replikativen freien Formen des Plasmids pSAM2 in den Actinomyceten ermöglichen kann,
c) den Varianten dieser Sequenz, die aus der Degeneration des genetischen Codes resultieren und für ein Polypeptid kodieren, das aus integrierten Formen das Auftreten von replikativen freien Formen des Plasmids pSAM2 in den Actinomyceten ermöglichen kann.

2. Expressionskassette, umfassend eine Sequenz gemäß Anspruch 1 unter Kontrolle eines konstitutiven oder regulierten Promotors.

3. Expressionskassette gemäß Anspruch 2, **dadurch gekennzeichnet, dass** der konstitutive Promotor ausgewählt ist unter dem Promotor des Gens ermE oder einer Variante von diesem, dem Promotor p14 der Phage I19 von S. ghanaensis oder jedem Fragment, das eine Promotorregion eines ribosomalen Operons von S. ambofaciens enthält.

4. Expressionskassette gemäß Anspruch 2, **dadurch gekennzeichnet, dass** der regulierte Promotor ausgewählt ist unter den Promotoren, die spezifisch durch ein Mittel induziert werden, das in das Kulturmedium eingeführt wird, wie beispielsweise der Promotor tipA, der durch Thiostrepton induzierbar ist, oder thermoinduzierbaren Promotoren, wie derjenige der Gene groEL.

5. Expressionskassette gemäß Anspruch 2, **dadurch gekennzeichnet, dass** der regulierte Promotor ausgewählt ist unter den Promotoren von Actinomyceten, die in den späten Phasen des Proliferationszyklus der Actinomyceten spezifisch aktiv sind, wie beispielsweise die Promotoren von Genen des sekundären Metabolismus (insbesondere Gene zur Produktion von Antibiotika).

6. Verwendung einer Sequenz oder Kassette gemäß einem der vorhergehenden Ansprüche zum Induzieren des Auftretens freier Kopien von Vektoren, die von pSAM2 abgeleitet sind, integriert in einen Actinomycet.

7. Verwendung gemäß Anspruch 6, **dadurch gekennzeichnet, dass** die Sequenz oder die Kassette durch den integrativen Vektor, der von pSAM2 abgeleitet ist, getragen werden.

8. Verwendung gemäß Anspruch 6, **dadurch gekennzeichnet, dass** die Sequenz oder die Kassette von einem anderen Vektor getragen werden oder direkt so, wie sie sind, eingeführt werden.

9. Verwendung gemäß den Ansprüchen 6 bis 8, **dadurch gekennzeichnet, dass** der Actinomycet ausgewählt ist unter den Streptomyces und den Mykobakterien.


### LISTE DE SEQUENCES

(1) INFORMATION GENERALE:
   (i) DEPOSANT:
      (A) NOM: RHONE-POULENC RORER S.A.
      (B) RUE: 20, avenue R. ARON
      (C) VILLE: ANTONY
      (E) PAYS: FRANCE
      (F) CODE POSTAL: 920165
   (ii) TITRE DE L' INVENTION: Séquences d'acides nucléiques régulatrices et utilisations.
   (iii) NOMBRE DE SEQUENCES: 1
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 812 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (ix) CARACTERISTIQUE ADDITIONELLE:
      (A) NOM/CLE: CDS
      (B) EMPLACEMENT: 155..505
      (D) AUTRES RENSEIGNEMENTS: /product= "gene rmf"
   (ix) CARACTERISTIQUE ADDITIONELLE:
      (A) NOM/CLE: -35_signal
      (B) EMPLACEMENT: 65..70
   (ix) CARACTERISTIQUE ADDITIONELLE:
      (A) NOM/CLE: -10_signal
      (B) EMPLACEMENT: 89..94
   (ix) CARACTERISTIQUE ADDITIONELLE:
      (A) NOM/CLE: promoter
      (B) EMPLACEMENT: 1..101
   (ix) CARACTERISTIQUE ADDITIONELLE:
      (A) NOM/CLE: RBS
      (B) EMPLACEMENT: 140..145
   (xi) DESCRIPTION DE LA SEQUENCE: SEQ ID NO: 1:
   CCAGCAGCCG ACTGACGACC GCTCAACTCC TCACAGCCCG TCGCGAGTTC TCTGTCGCGG    60
   CGGGTTGACT CATGTATAGG AGTGGTGCAC TCTTCTTCAT GTCACTCATA TACATGAGTG   120
   ACGGAGTCCA GCCTCTATAG AGGAGTGATC CGCT GTG CGT CAG ATC CCC GTC       172
                                         Met Arg Gln Ile Pro Val
                                          1               5
   GAC ACC TCC GCC GCA ACC GTG ATG GTC GCC AAG ACT CCG GAG CCG AAG     220
   Asp Thr Ser Ala Ala Thr Val Met Val Ala Lys Thr Pro Glu Pro Lys
               10                  15                  20
   GTG AAG GAC CGC CGG ACC GGT GAG CTG GCC GTC GAC GCC GAG ACC GGT     268
   Val Lys Asp Arg Arg Thr Gly Glu Leu Ala Val Asp Ala Glu Thr Gly
           25                  30                  35
   GCC AAG CTC ATG ACC GTG AAC GTG ATG TTC GCG GCC AAC GAC GAA GTC     316
   Ala Lys Leu Met Thr Val Asn Val Met Phe Ala Ala Asn Asp Glu Val
       40                  45                  50
   GAG ATT CTG TCC GTG ACC GTC CCG GAG ACC GGT ATC TCC GGT GAA CTG     364
   Glu Ile Leu Ser Val Thr Val Pro Glu Thr Gly Ile Ser Gly Glu Leu
   55                  60                  65                  70
   GCC ATG GGT ACC CCG GTC GCG CTG ACG GGG CTC ATC GCC CGG CCG TGG     412
   Ala Met Gly Thr Pro Val Ala Leu Thr Gly Leu Ile Ala Arg Pro Trp
                   75                  80                  85
   GAG AAC GAG TTC AAC GGC CAG AAG CGG CAC GGC ATC GCG TTC CGC GCG     460
   Glu Asn Glu Phe Asn Gly Gln Lys Arg His Gly Ile Ala Phe Arg Ala
               90                  95                 100
   GTC GCG GTC ACG TCG CTG ACC GCT GCG GGC TCG AAG GCT GCC TGATCATGAC  512
   Val Ala Val Thr Ser Leu Thr Ala Ala Gly Ser Lys Ala Ala
           105                 110                 115
   GTGGTTCATG GTCGCTGTGG TTGTGGTCGT CGCTGCTGCG GGTCTCCTGC GGTGGCGGCG   572
   CCCCGCCTGG TACTGGCTCA CCTTCGGGGC CCTGGTCGCG ACGGTGCGGG TCCTGGTCCG   632
   TACGCCTCGG TCATGGAAGC GTGCGGGCTG ACGGTCCGCC CTCACGCTGG CGGCTGCTCT   692
   GGCCCGGATG GCGAATGCCG CGCCTGAGTC CCGGCCGCCG CGCATCTTGC GGTTACGTCC   752
   CACTCGTACC GGCCTGGTCT GCGGCTCAAG CTCCGGCCGG GACAGGATGC CTTCGACGTG   812